# EUROPEAN PATENT SPECIFICATION

(11) **EP 2 125 804 B1**
(45) Date of publication and mention of the grant of the patent: **26.01.2011**
(21) Application number: 07846763.6
(22) Date of filing: 23.11.2007
(51) Int. Cl.: C07D 471/04, A61K 31/437, A61P 11/06, A61P 37/08, A61P 9/12, A61P 9/10, A61P 29/00, A61P 3/10, A61P 37/00

(54) **5-PHENYL-6-PYRIDIN-4-YL-1,3-DIHYDRO-2H-IMIDAZO[4,5-B]PYRIDIN-2-ONE DERIVATIVES USEFUL AS A2B ADENOSINE RECEPTOR ANTAGONISTS**
ALS A2B-ADENOSINREZEPTORANTAGONISTEN GEEIGNETE 5-PHENYL-6-PYRIDIN-4-YL-1,3-DIHYDRO-2H-IMIDAZO[4,5-B]PYRIDIN-2-ONDERIVATE
DÉRIVÉS DE 5-PHÉNYL-6-PYRIDIN-4-YL-1,3-DIHYDRO-2H-IMIDAZO[4,5-B]PYRIDIN-2-ONE UTILISABLES EN TANT QU'ANTAGONISTES DU RÉCEPTEUR A2B DE L'ADÉNOSINE

(30) Priority: 29.12.2006 ES 200603309
(43) Date of publication of application: 02.12.2009
(73) Proprietor: Almirall, S.A., 08022 Barcelona (ES)
(72) Inventor: AIGUADE BOSCH, Jose, E-08025 Barcelona (ES); CARRANCO MORUNO, Ines, E-08690 Sta. Coloma de Cervello (ES); VIDAL JUAN, Bernat, E-08396 St. Cebria de Vallalta (ES)
(74) Representative: Winkler, Andreas Fritz Ernst
(86) International application number: PCT/EP2007/010162
(87) International publication number: WO 2008/080461

(56) References cited:
- WO-A-2007/039297
- WO-A1-2005/100353

## Description

The present invention relates to new antagonists of the A_{2B} adenosine receptor. These compounds are useful in the treatment, prevention or suppression of diseases and disorders known to be susceptible to improvement by antagonism of the A_{2B} adenosine receptor, such as asthma, allergic diseases, inflammation, atherosclerosis, hypertension, gastrointestinal tract disorders, cell proliferation disorders, diabetes mellitus and autoimmune diseases. These compounds are also useful in the treatment, prevention or suppression of diseases and disorders which are also known to be susceptible to improvement by antagonism of the A_{2B} adenosine receptor such as hepatic disease and wounds.

Adenosine regulates several physiological functions through specific cell membrane receptors, which are members of the G-protein coupled receptor family. Four distinct adenosine receptors have been identified and classified: A₁, A_{2A}, A_{2B} and A₃.

The A_{2B} adenosine receptor subtype (see Feoktistov, I., Biaggioni, I. Pharmacol. Rev. 1997, 49, 381-402) has been identified in a variety of human and murine tissues and is involved in the regulation of vascular tone, smooth muscle growth, angiogenesis, hepatic glucose production, bowel movement, intestinal secretion, and mast cell degranulation.

In view of the physiological effects mediated by adenosine receptor activation, several A_{2B} receptor antagonists have been recently disclosed for the treatment or prevention of, asthma, bronchoconstriction, allergic diseases, hypertension, atherosclerosis, reperfusion injury, myocardial ischemia, retinopathy, inflammation, gastrointestinal tract disorders, cell proliferation diseases and/or diabetes mellitus. See for example WO03/063800, WO03/042214, WO 03/035639, WO02/42298, EP 1283056, WO 01/16134, WO 01/02400, WO01/60350, WO 00/73307 or WO 2005/100353.

It has now been found that certain imidazopyridinone derivatives are novel potent antagonists of the A_{2B} adenosine receptor as well as very selective against A₁, A_{2A} and A₃ adenosine receptors subtypes and can therefore be used in the treatment or prevention of these diseases.

Selectivity *versus* the A₁ receptor is required to avoid any side effects resulting from blockade of this receptor like central nervous system stimulation, gastric secretion, diuresis and arrythmias (Fozard JR & mccarthy C. currr Opin Invest Drugs 2002, 3(1): 69-77*;* Barnes P. Am J Respir Crit Care Med 2003, 167: 813-818).

Ensuring selectivity versus the A_{2A} adenosine receptor is important in the context of asthma due to the reported anti-inflammatory effects mediated by this receptor (*reviewed in* Lappas CM, Sullivan GW, Linden J. Expert Opin Investig Drugs. 2005, 14(7):797-806), while selectivity *versus* the A₃ receptor avoids interference with its potential roles in heart protection and tumor prevention (*reviewed in* Jacobson KA & Zhan-Guo G. Nature Reviews 2006, 5: 247-264).

Further objectives of the present invention are to provide a method for preparing said compounds; pharmaceutical compositions comprising an effective amount of said compounds; the use of the compounds in the manufacture of a medicament for the treatment of pathological conditions or diseases susceptible to improvement by antagonism of the A_{2B} adenosine receptor.

Thus, the present invention is directed to new imidazopyridinone derivatives of formula (I) 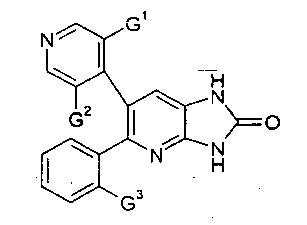 wherein G¹ is selected from the groups consisting of fluorine and chlorine atoms, G² is selected from the groups consisting of hydrogen, fluorine and chlorine atoms and G³ is selected from the groups consisting of fluorine and chlorine atoms
and pharmaceutically acceptable salts or N-oxides thereof.

As used herein, the term pharmaceutically acceptable salt embraces salts with a pharmaceutically acceptable acid. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or *p*-toluenesulphonic acid.

As used herein, an N-oxide is formed from the tertiary basic amines or imines present in the molecule, using a convenient oxidising agent.

In an embodiment of the present invention G³ is a fluorine atom.

In another embodiment of the present invention G² is a fluorine atom.

In a still more preferred embodiment of the present invention G¹ is a fluorine atom.

Particular individual compounds of the invention include:
5-(2-Fluorophenyl)-6-(3-fluoropyrdin-4-yl)-1,3-dihydro-2*H*-imidazo[4,5-*b*]pyridin-2-one
6-(3-Chloropyrdin-4-yl)-5-(2-fluorophenyl)-1,3-dihydro-2*H*-imidazo[4,5-*b*]pyridin-2-one
6-(3,5-Difluoropyrdin-4-yl)-5-(2-fluorophenyl)-1,3-dihydro-2*H*-imidazo[4,5-*b*]pyridin-2-one
6-(3,5-Difluoropyridin-4-yl)-5-(2-fluorophenyl)-1,3-dihydro-2*H*-imidazo[4,5-*b*]pyridin-2-one hydrochloride
6-(3,5-Difluoropyridin-4-yl)-5-(2-fluorophenyl)-1,3-dihydro-2*H*-imidazo[4,5-*b*]pyridin-2-one (tosylate salt)

Compounds of general formula (I) may be prepared following the synthetic scheme depicted in figure 1.

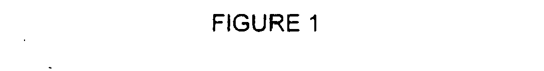

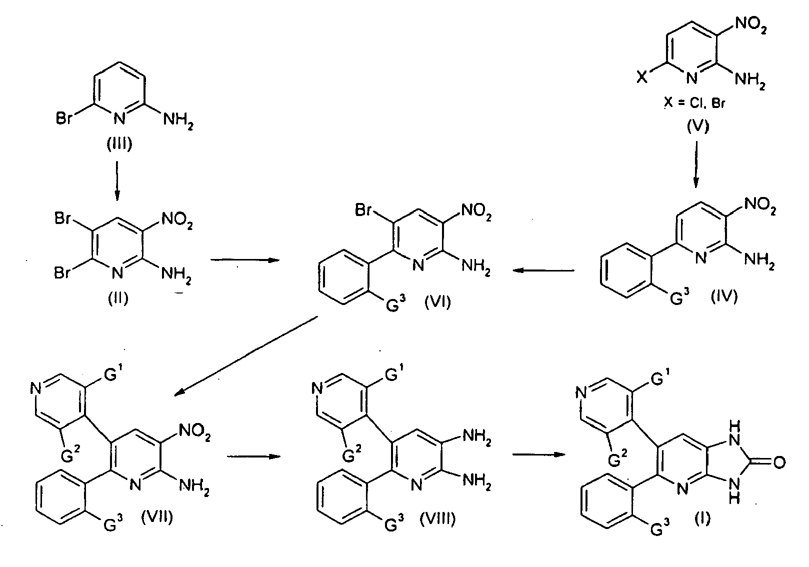

Compounds of general formula (VIII) are prepared in several steps starting with the halogenation of 6-halopyridine derivatives (III) using reagents such as bromine or N-halosuccinimide in polar aprotic solvents such as DMF and at temperatures ranging from 0°C to 100°C, to yield 5,6-dihalo-2-aminopyridines (not shown). These products are in turn nitrated in a two step process involving nitration of the amino group in a mixture of sulphuric and nitric acid in a temperature range between -10°C and 0 °C followed by a sulphuric acid promoted rearrangement of the nitro group to produce compounds of formula (II).

Regioselective Suzuki-type coupling of (II) with a boronic acid or boronate derivative using a palladium catalyst such as tetrakis(triphenylphosphine)palladium(0) or [1,1'-bis(diphenylphosphino)ferrocene] palladium(II)dichloride dichloromethane complex (1:1) in solvents such as toluene or dioxane in the presence of an aqueous solution of a base such as sodium or caesium carbonate and at a temperature between 25°C and 110°C provides compounds of general formula (VI).

Compounds of general formula (IV) are prepared from compounds of general formula (V) using the general Suzuki coupling procedure described above. Bromination using similar conditions as used in the preparation of (II) provides compounds of general formula (VI).

A further Suzuki-type coupling using compounds of formula (VI) with a corresponding boronic acid or boronate derivative under the standard procedures for Pd catalyzed reactions described above provides the 2-amino-3-nitropyridines of formula (VII).

alternatively, Stille-type cross coupling of bromopyridine of formula (VI) with a corresponding organotin derivative in the presence of palladium catalysts such as tetrakis(triphenylphosphine) palladium (0) in solvents such as xylene or dimethylformamide at a temperature between 25°C to 200°C also provides compounds of general formula (VII).

In the particular case where G¹ and G² are fluorine atoms, compounds of general formula (VII) can be prepared by Negishi-type cross coupling of bromopyridine (VI) using the organozinc derivative of 3,5-difluoropyridine in the presence of palladium catalysts such as tetrakis(triphenylphosphine)palladium (0) in solvents such as tetrahydrofuran at a temperature between 25°C to 180°C.

Reduction of the nitro group using standard hydrogenation conditions in the presence of hydrogen and using palladium on carbon as a catalyst provides the diamino derivatives of general formula (VIII).

Treatment of derivatives of general formula (VIII) with carbonylating agents such as carbonyldiimidazole in polar aprotic solvents such as dimethylformamide and heating at temperatures between 50°C and 200°C provides the imidazopyridinone compounds of general formula (I).

Compounds of general formula (I) may also be prepared following the synthetic scheme depicted in figure 2.

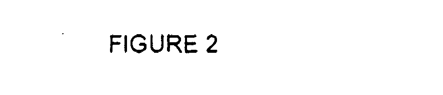

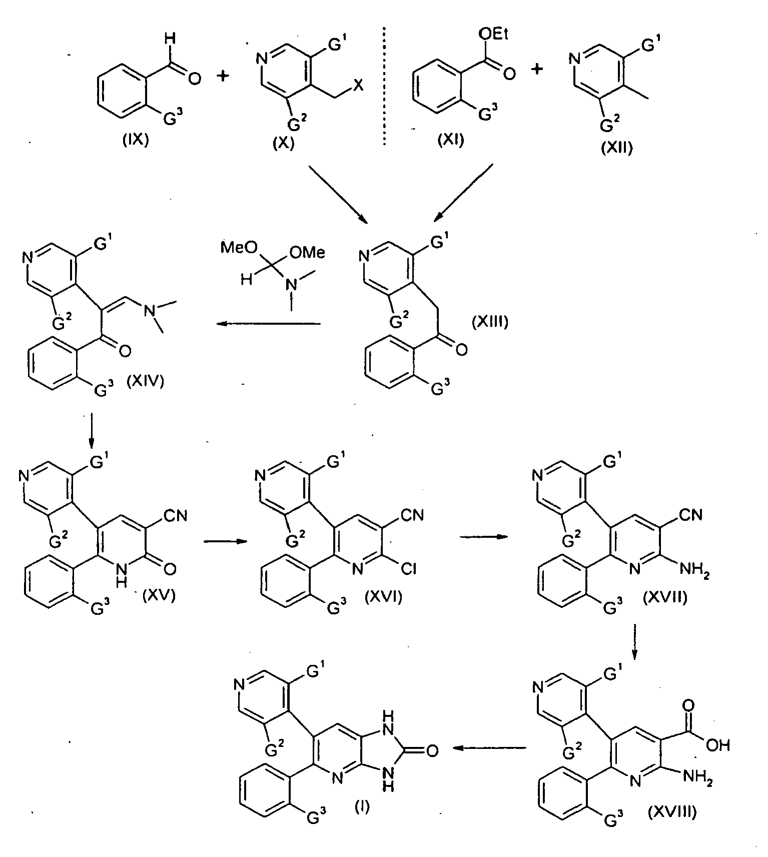

The aldehydes of formula (IX) are reacted with the halomethyl derivatives of formula (X) to yield ketones of formula (XIII) either via cyanohydrin intermediates or in a two step process involving the addition of an organometallic derivative of (X), preferably a magnesium or zinc derivative, followed by oxidation of the resulting alcohol using oxidizing agents such as manganese (IV) oxide.

Alternatively ketones of formula (XIII) may be obtained by condensation of ethyl esters of formula (XI) with compounds of formula (XII). This reaction is conveniently carried out in the presence of an organic base such as lithium bis(trimethylsilyl)amide at temperatures ranging from -10°C to about 50°C in an organic aprotic solvent, preferably tetrahydrofuran or diethyl ether.

Ketones of formula (XIII) may be reacted with neat N,N-dimethylformamide dialkyl acetal, such as dimethylacetal, at a temperature ranging from room temperature to 150°C to yield dimethylamino α,β unsaturated ketones of formula (XIV) which can be converted into the 2-oxo-1,2-dihydropyridine-3-carbonitriles of formula (XV) by cyclization in the presence of cyanoacetamide using alkoxides such as sodium methoxide in polar aprotic solvents such as dimethylformamide and at temperatures ranging from 50°C to 150°C. These compounds may be converted into the 2-chloronicotinonitriles of formula (XVI) by treatment of the resulting pyridone (XV) with chlorinating agents such as POCl₃, PCl₅ or PhPOCl₂ or by using a combination of such reagents.

2-Chloronicotinonitriles of formula (XVI) may be reacted with a saturated solution of ammonia in an organic solvent, preferably ethanol, at a temperature ranging from 25°C to 150°C to yield compounds of formula (XVII). Hydrolisis of compounds (XVII) to the carboxylic acid of formula (XVIII) can be achieved with a base such as potassium hydroxyde in aqueous or organic solvents such as ethylene glycol and at a temperature between 50°C and 200°C. Alternatively this conversion can be achieved by heating (XVII) in an aqueous acidic medium such as 6M aqueous sulphuric acid. Compounds (XVIII) may be subjected to Curtius rearrangement by formation of an acyl azide using reagents such as diphenylphosphoryl azide (or sodium azide with activated acid) in an organic solvent compatible with these reaction conditions (e.g. dioxane) then heating the reaction mixture at a temperature between 50°C and 200°C, with *in situ* formation of the target imidazopyridinone ring yielding compounds of formula (I).

Alternative general synthetic methods are depicted in figure 3.

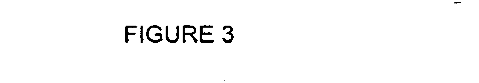

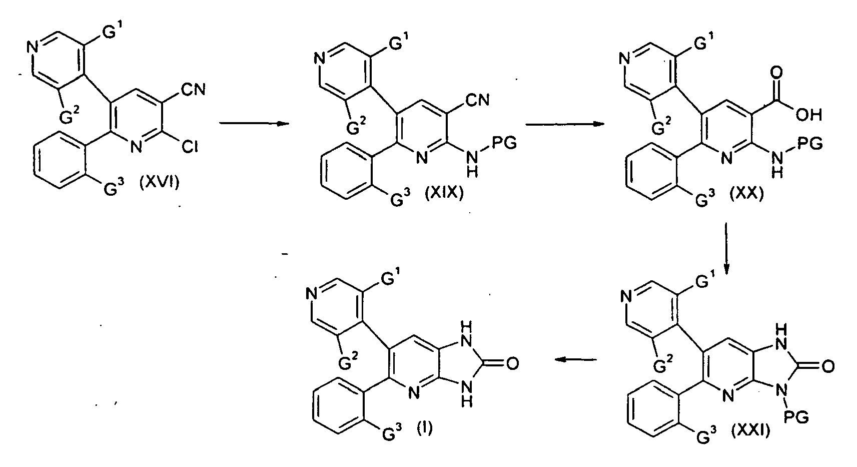

Cyanopyridine (XVI) reacts with conveniently protected amines, such as 4-methoxybenzylamine or 2,4-dimethoxybenzylamine, in the presence of a base such as triethylamine in a suitable solvent such as ethanol with or without the influence of microwave irradiation at temperatures ranging from 60-200 °C to give substituted derivatives of type (XIX). Hydrolisis of compounds (XIX) to the carboxylic acid of formula (XX) can be achieved with a base such as potassium hydroxide in aqueous or organic solvents such as ethylene glycol and at a temperature ranging from 50 °C to 200 °C. These compounds may be subjected to Curtius rearrangement by formation of an acyl azide using reagents such as diphenylphosphoryl azide (or sodium azide with activated acid) in an organic solvent compatible with these reaction conditions (e.g. dioxane) then heating the reaction mixture at a temperature between 50°C and 200°C, with in situ formation of the target imidazopyridinone ring yielding compounds of formula (XXI). Treatment of compounds of type (XXI) with a suitable base, such as sodium hydride or potassium carbonate, in a polar aprotic solvent, such as dimethylformamide or dimethylsulfoxide, followed by the addition of an alkylating agent such as an alkyl bromide or iodide followed by removal of the amine protecting group by using, for example, an acid such as trifluoroacetic acid in the presence of a cation scavenger such as thioanisole at temperatures ranging from 0-100 °C gives rise to molecules of type (I).

The imidazopyridinones of formula (I) may be converted into salts with different pharmaceutically acceptable anions by mixing a solutiuon of the imidazopyridinone free base in dioxane with the acid corresponding to the anion and stirring the mixture for a time period of 0, 5 to 4 hours. The mixture is then diluted with diethylether and filtered. The solid is dried over solid CaSO₄ under vacuum for 8 to 24 h.

### Radioligand binding assays:

For A₁ receptors a filtration binding assay was performed with 2 nM ³H-DPCPX, 100 mM unlabelled R-PIA, membranes from CHO cells transfected with human A₁ receptor (Euroscreen ES-010-C) and incubated 90 min. at room temperature in Hepes 20 mM pH 7.4, NaCl 100 mM, MgCl₂ 10 mM and 2 U/ml adenosin deaminase.

For A_{2A} receptors binding technology was SPA (Amhersham) with 3,3 nM ³H-ZM241385, 50 mM unlabelled NECA, membranes from HeLa cells transfected with human A_{2A} receptor, incubated 1h. at room temperature with 1 mg YSi-WGA beads in TrisHCl 50 mM pH 7.4, EDTA 1 mM, MgCl₂ 10 mM, 2 U/ml adenosin deaminase.

For A_{2B}, competition assays were carried out in filtration binding assay, incubating in polypropylene 96 well-plates (n° 267245, NUNC) containing 2 µL of either 1% DMSO solution, test compound or 100 µM 5'NECA (SIGMA E-2387) for non-specific binding, 100 µg of A_{2B}-membranes prepared from HEK293 cells stably expressing the human A_{2B} receptor (Euroscreen ES-013-C) and 35 nM [³H]-DPCPX (TRK1064, 128Ci/mmol, Amersham), in a total volume of 200 µl of buffer A + 2UI/ml adenosine deaminase, for 60 minutes at room temperature.

For A₃ receptors, in filtration binding assay, 30 nM ³H-NECA, 100 mM unlabelled R-PIA, 100 mg membranes from HeLa cells transfected with human A₃ receptor, incubated 3 h. at room temperature in TrisHCl 50 mM pH 7.4, EDTA 1 mM, MgCl₂ 5 mM, 2 U/ml adenosin deaminase.

The compounds of formula (I) have been tested according to the assay described above and have shown to be extremely potent inhibitors of the A_{2B} adenosine receptor subtype which possess a functional Kᵢ value for the inhibition of A_{2B} (determined as defined above) of less than 2,0 nM. They have also shown a high selectivity over other adenosine receptor subtypes such as the A₁ adenosine receptor, the A_{2A} adenosine receptor and the A₃ adenosine receptor.

**TABLE 1**

| EXAMPLE | Kᵢ^{b} (nM) or % inhibition of radioligand binding at indicated concentration | | | |
|---|---|---|---|---|
| | hA_{2B} | hA_{2A} | hA₁ | hA₃ |
| **1** | 0.9 | 721 | 35 | >1000(19%) |
| **2** | 1.8 | 449 | 76 | >1000(22%) |
| **3** | 1.1 | >2500 (6%) | 632 | >1000(18%) |

| | | | | |
|---|---|---|---|---|
| ^{b}Kᵢ values are reported as the mean of at least two independent determinations. | | | | |

The imidazopyridinone derivatives of the invention are useful in the treatment or prevention of diseases known to be susceptible to improvement by treatment with an antagonist of the A_{2B} adenosine receptor. Such diseases are, for example, asthma, bronchoconstriction, allergic diseases, inflammation, reperfusion injury, myocardial ischemia, atherosclerosis, hypertension, retinopathy, diabetes mellitus, inflammation, gastrointestinal tract disorders, and/or autoimmune diseases. Examples of autoimmune diseases which can be treated or prevented using the compounds of the invention are Addison's disease, autoimmune hemolytic anemia, Crohn's disease, Goodpasture's syndrome, Graves disease, Hashimoto's thyroiditis, idiopathic thrombocytopenic purpura, insulin-dependent diabetes mellitus, multiple sclerosis, myasthenia gravis, pemphigus vulgaris, pernicious anemia, poststreptococcal glomerulonephritis, psoriasis, rheumatoid arthritis, scleroderma, Sjogren's syndrome, spontaneous infertility, and systemic lupus erythematosus.

Accordingly, the imidazopyridinone derivatives of the invention and pharmaceutically acceptable salts thereof, and pharmaceutical compositions comprising such compound and/or salts thereof, may be used in a method of treatment of disorders of the human or animal body which comprises administering to a subject requiring such treatment an effective amount of imidazopyridinone derivative of the invention or a pharmaceutically acceptable salt thereof.

The present invention also provides pharmaceutical compositions which comprise, as an active ingredient, at least a imidazopyridinone derivative of formula (I) or a pharmaceutically acceptable salt thereof in association with a pharmaceutically acceptable excipient such as a carrier or diluent. The active ingredient may comprise 0.001 % to 99% by weight, preferably 0.01 % to 90% by weight of the composition depending upon the nature of the formulation and whether further dilution is to be made prior to application. Preferably the compositions are made up in a form suitable for oral, topical, nasal, rectal, percutaneous injectable administration or inhalation.

The pharmaceutically acceptable excipients which are admixed with the active compound or salts of such compound, to form the compositions of this invention are well-known *per se* and the actual excipients used depend *inter alia* on the intended method of administering the compositions.

Compositions of this invention are preferably adapted for injectable and oral administration. In this case, the compositions for oral administration may take the form of tablets, retard tablets, sublingual tablets, capsules, inhalation aerosols, inhalation solutions, dry powder inhalation, or liquid preparations, such as mixtures, elixirs, syrups or suspensions, all containing the compound of the invention; such preparations may be made by methods well-known in the art.

The diluents which may be used in the preparation of the compositions include those liquid and solid diluents which are compatible with the active ingredient, together with colouring or flavouring agents, if desired. Tablets or capsules may conveniently contain between 2 and 500 mg of active ingredient or the equivalent amount of a salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Effective doses are normally in the range of 2-2000 mg of active ingredient per day. Daily dosage may be administered in one or more treatments, preferably from 1 to 4 treatments, per day.

The syntheses of the compounds of the invention and of the intermediates for use therein are illustrated by the following Examples (1 to 3) including Preparation Example 1 which do not limit the scope of the invention in any way.

¹H Nuclear Magnetic Resonance Spectra were recorded on a Varian Gemini 300 spectrometer. The chromatographic separations were obtained using a Waters 2795 system equipped with a Symmetry C18 (2.1 x 100 mm, 3.5 mm) column. As detectors a Micromass ZMD mass spectrometer using ES ionization and a Waters 996 Diode Array detector were used. The mobile phase was formic acid (0.46 ml), ammonia (0.115 ml) and water (1000 ml) (A) and formic acid (0.4 ml), ammonia (0.1 ml), methanol (500 ml) and acetonitrile (500 ml) (B): initially from 0% to 95% of B in 20 min, and then 4 min. with 95% of B. The reequilibration time between two injections was 5 min. The flow rate was 0.4 ml/min. The injection volume was 5 µl. Diode array chromatograms were processed at 210 nm.

### PREPARATION EXAMPLES

### INTERMEDIATE 1

### 5-Bromo-6-(2-fluorophenyl)-3-nitropyridin-2-amine

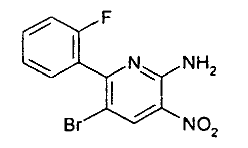

### Step 1: 6-(2-Fluorophenyl)-3-nitropyridin-2-amine

An oven dried resealable Schlenk tube was charged with 6-chloro-3-nitropyridin-2-amine (5.00 g, 28.81 mmol), (2-fluorophenyl)boronic acid (6.05 g, 43.22 mmol), dioxane (288 mL) and a 2M aqueous solution of cesium carbonate (43.22 mL, 86.43 mmol). The Schlenk tube was subjected to three cycles of evacuation-backfilling with argon, and 1,1'-bis(diphenylphosphino)ferrocene-palladium(II) dichloride dichloromethane complex (1.41 g, 1.73 mmol) was added. After three new cycles of evacuation-backfilling with argon, the Schlenk tube was capped and placed in a 90°C oil bath. After 16h, the mixture was cooled and the solvent was evaporated. The crude residue was purified by silica gel flash chromatography (3:1 hexane/ethyl acetate) to give the title compound (5.59 g, 83 %) as a yellow solid.
δ ¹H-NMR (CDCl₃): 8.48 (d, 1H), 7.99 (dt, 1H), 7.52-7.49 (m, 1H), 7.32-7.12 (m, 3H), 1.60 (s, 2H),
ESI/MS m/e: 234 ([M+H]⁺, C₁₁H₈FN₃O₂)

### Step 2: 5-Bromo-6-(2-fluorophenyl)-3-nitropyridin-2-amine

To a 0°C cooled stirred solution of 6-(2-fluorophenyl)-3-nitropyridin-2-amine (0.50 g, 2.15 mmol) in DMF (11 mL), N-bromosuccinimide (0.42 g, 2.35 mmol) was added in portions. After stirring at room temperature for 16h, the solution was poured into water and ice. The precipitate formed was filtered off, washed with water and dried to give the title compound (0.58 g, 86%) as a yellow solid.
δ ¹H-NMR (CDCl₃): 8.70 (s, 1H), 7.55-7.16 (m, 4H), 1.60 (s, 2H).
ESI/MS m/e: 312 ([M+H]⁺, C₁₁H₇BrFN₃O₂)

### EXAMPLES

### EXAMPLE 1

### 5-(2-Fluorophenyl)-6-(3-fluoropyridin-4-yl)-1,3-dihydro-2H-imidazo[4,5-b]pyridin-2-one

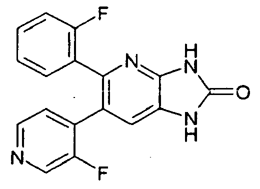

### Step A. 3'-Fluoro-2-(2-fluorophenyl)-5-nitro-3,4'-bipyridin-6-amine

A mixture of 5-bromo-6-(2-fluorophenyl)-3-nitropyridin-2-amine (Intermediate 1) (1 g, 3.20 mmol), 3-fluoro-4-(tributylstannyl)pyridine (1.36 g, 3.52 mmol), bis(triphenylphosphino) palladium (II) chloride (0.23 g, 0.32 mmol) and copper (I) iodide (0.12 g, 0.64 mmol) in dioxane (11 mL) was heated at 180 °C for 1 hour in Biotage Initiator Microwave Synthesizer.

The mixture was filtered through Celite® and the filter cake was washed with dioxane. The solvent was evaporated and the crude residue was purified by silica gel flash chromatography (2:1 hexane/ethyl acetate) to give the title compound (1.62 g, 38%) as a yellow solid.
δ ¹H-NMR (CDCl₃): 8.55 (s, 1H), 8.40 (d, 1H), 8.31 (d, 1H), 7.49-7.32 (m, 2H), 7.20 (dt, 1H), 7.04 (dd, 1H), 6.91 (ddd, 1H), 1.60 (s, 2H).
ESI/MS m/e: 329 ([M+H]⁺, C₁₆H₁₀F₂N₄O₂)

### Step B. 3'-Fluoro-2-(2-fluorophenyl)-3,4'-bipyridine-5,6-diamine

A suspension of 3'-fluoro-2-(2-fluorophenyl)-5-nitro-3,4'-bipyridin-6-amine (1.62 g, 4.93 mmol) and 20% palladium on carbon (0.32 g) in ethanol (55 mL) was stirred under hydrogen atmosphere. After 3h, the mixture was filtered through Celite® and the filter cake was washed with ethanol. The combined filtrate and washings were evaporated to give the title compound as a solid (1.41 g, 96%).
δ ¹H-NMR (CD₃OD): 8.27 (d, 1H), 8.15 (dd, 1H), 7.35-7.22 (m, 2H), 7.11 (s, 1H), 7.16-7.08 (m, 1H), 6.99 (d, 1H), 6.95-6.86 (m, 1H).
ESI/MS m/e: 299 ([M+H]⁺, C₁₆H₁₂F₂N₄)

### Step C. 5-(2-Fluorophenyl)-6-(3-fluoropyridin-4yl)-1,3-dihydro-2H-imidazo[4,5-b]pyridin-2-one

To a solution of 3'-fluoro-2-(2-fluorophenyl)-3,4'-bipyridine-5,6-diamine (46 mg, 0.15 mmol) in THF (1 mL) Et₃N (42 µL, 0.30 mmol) and carbonyldiimidazole (49 mg, 0.30 mmol) were added sequentially. The reaction mixture was heated at 80°C. After 18 h the solvent was removed under reduced pressure and the crude residue was purified by silica gel flash chromatography (95:5 CH₂Cl₂/MeOH) to give the title compound (35 mg, 71 %) as a solid.
δ ¹H-NMR (CD₃OD): 8.35 (d, 1H), 8.23 (dd, 1H), 7.70 (bs, 1H), 7.42 (s, 1H), 7.37 (m, 3H), 7.20 (m, 1H), 7.09 (dd, 1H), 6.92 (ddd, 1H).
ESI/MS m/e: 325 ([M+H]⁺, C₁₇H₁₀F₂N₄O)

### EXAMPLE 2

### 6-(3-Chloropyridin-4-yl)-5-(2-fluorophenyl)-1,3-dihydro-2H-imidazo[4,5-b]pyridin-2 one

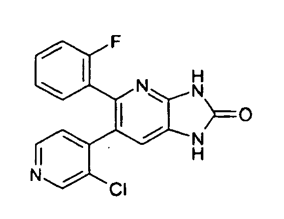

### Step A. 3'-Chloro-2-(2-fluorophenyl)-5-nitro-3,4'-bipyridin-6-amine

An oven-dried resealable Schlenk tube was charged with 5-bromo-6-(2-fluorophenyl)-3-nitropyridin-2-amine (Intermediate 1) (200 mg, 0.64 mmol), 3-chloro-4-(4,4,5,5-tetramethyl-1,3,2-dioxaborolan-2-yl)pyridine (460 mg, 1.92 mmol), dioxane (6.4 mL) and a 2M aqueous solution of cesium carbonate (0.96 mL, 1.92 mmol). The Schlenk tube was subjected to three cycles of evacuation-backfilling with argon, and 1,1'-bis(diphenylphosphino)ferrocene-palladium(II) dichloride dichloromethane complex [PdCl₂dppf.DCM] (0.052 g, 0.06 mmol) was added. After three new cycles of evacuation-backfilling with argon, the Schlenk tube was sealed and the mixture was stirred and heated in an oil bath to 95°C. After 20 hours, the mixture was cooled and filtered through Celite® and the filter cake was washed with dioxane. The solvent was removed under reduced pressure and the crude residue was solved with ethyl acetate and washed with water. The organic layer was washed with brine and evaporated. The residue was purified by silica gel flash chromatography (3:2 hexane/ethyl acetate) to give the title compound (120 mg, 54%) as a solid.
δ ¹H-NMR (CDCl₃): 8.57 (s, 1H) 8.51 (s, 1H), 8.37 (d, 1H), 7.44-7.28 (m, 2H), 7.16 (t, 1H), 7.01 (dd, 1H), 6.90 (t, 1H), 1.26 (s, 1H)
ESI/MS m/e: 345 ([M+H]⁺, C₁₆H₁₀CIFN₄O₂)

### Step B. 3'-Chloro-2-(2-fluorophenyl)-3,4'-bipyridine-5,6-diamine

3'-Chloro-2-(2-fluorophenyl)-5-nitro-3,4'-bipyridin-6-amine (119 mg, 0.35 mmol) was dissolved in EtOH (3.5 mL) and conc. HCl (220 µL). Iron metal (98 mg, 1.75 mmol) was added to the suspension and the mixture was heated to 90 °C for 2 h. The suspension was then filtered through Celite^{®} and the solvent removed *in vacuo.* NaHCO₃ (20 mL of a 4% w/w aqueous solution) was added to the residue and the aqueous phase was extracted with AcOEt (3 x 20 mL). The organic layer was washed with brine and evaporated. The residue was purified by silica gel flash chromatography (ethyl acetate/TEA 1%) to give the title compound (44 mg, 40%) as a solid.
δ ¹H-NMR (CD₃OD): 8.44 (s, 1H), 8.22 (d, 1H), 7.31-7.21 (m, 2H), 7.12-7.03 (m, 2H), 6.92 (s, 1H), 6.94-6.85 (m, 1H).
ESI/MS m/e: 315 ([M+H]⁺, C₁₆H₁₂CIFN₄)

### Step C. 6-(3-Chloropyridin-4-yl)-5-(2-fluorophenyl)-1,3-dihydro-2H-imidazo[4,5-b]pyridin-2-one

To a solution of 3'-chloro-2-(2-fluorophenyl)-3,4'-bipyridine-5,6-diamine (44 mg, 0.14 mmol) in THF (1 mL) Et₃N (39 µL, 0.28 mmol) and carbonyldiimidazole (45 mg, 0.28 mmol) were added sequentially. The reaction mixture was heated at 80°C. After 18 h the solvent was removed under reduced pressure and the crude residue was purified by silica gel flash chromatography (95:5 CH₂Cl₂/MeOH) to give the title compound (40 mg, 83%) as a solid.
δ ¹H-NMR (CD₃OD): 8.50 (s, 1H), 8.29 (dd, 1H), 7.69 (bs, 1H), 7.35 (s, 1H), 7.40-7.25 (m, 2H), 7.21 (d, 1H), 7.15-7.07 (m, 1H), 7.06 (dd, 1H), 6.97-6.87 (m, 1H).
ESI/MS m/e: 341 ([M+H]⁺, C₁₇H₁₀CIFN₄O)

### EXAMPLE 3

### 6-(3,5-Difluoropyridin-4-yl)-5-(2-fluorophenyl)-1,3-dihydro-2H-imidazo[4,5-b]pyridin-2-one

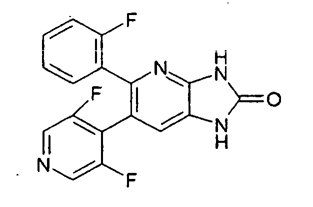

### Step A. 3',5'-Difluoro-2-(2-fluorophenyl)-5-nitro-3,4'-bipyridin-6-amine

A mixture of 5-bromo-6-(2-fluorophenyl)-3-nitropyridin-2-amine (Intermediate 1) (0.90 g, 2.88 mmol), 3,5-difluoro-4-tributylstannanylpyridine (1.16 g, 2.88 mmol), bis(triphenylphosphino) palladium (II) chloride (0.20 g, 0.29 mmol) and copper (I) iodide (0.11 g, 0.58 mmol) in dioxane (15 mL) was heated at 150°C for 6 hours in Biotage Initiator Microwave Synthesizer. The mixture was filtered through Celite® and the filter cake was washed with dioxane. The solvent was evaporated and the crude residue was purified by silica gel flash chromatography (8:2 hexane/ethyl acetate) to give the title compound (0.53 g, 53%) as a yellow.
δ ¹H-NMR (CDCl₃): 8.54 (s, 1H), 8.30 (s, 2H), 7.49-7.44 (m, 1H), 7.41-7.34 (m, 1H), 7.23-7.18 (m, 1H), 6.91 (t, 1H), 1.66 (s, 2H),
ESI/MS m/e: 347 ([M+H]⁺, C₁₆H₉F₃N₄O₂)

### Step B. 3',5'Difluoro-2-(2-fluorophenyl)-3,4'-bipyridine-5,6-diamine

3',5'-Difluoro-2-(2-fluorophenyl)-5-nitro-3,4'-bipyridin-6-amine (0.55 g, 1.59 mmol) was dissolved in EtOH (10 mL) and conc. HCl (2 mL). Tin (II) chloride dihydrate (1.25 g, 5.55 mmol) was added to the suspension and the mixture was heated to 80°C for 3 h. The pH was adjusted to 10 with solid sodium hydroxide 6N and EtOH was removed *in vacuo.* H₂O was added to the crude and the aqueous phase was extracted with CH₂Cl₂. The organic layer was dried, filtered and concentrated to dryness to yield the title compound (0.45g, 90%), which was used without further purification.
δ ¹H-NMR (CD₃OD): 8.21 (s, 2H), 7.34-7.23 (m, 2H), 7.10 (t, 1H), 6.94 (s, 1H), 6.89 (t, 1H).
ESI/MS m/e: 317 ([M+H]⁺, C₁₆H₁₁F₃N₄)

### Step C. 6-(3,5-Difluoropyridin-4-yl)-5-(2-fluorophenyl)-1,3-dihydro-2H-imidazo[4,5-b]pyridin-2-one

To a solution of 3',5'-difluoro-2-(2-fluorophenyl)-3,4'-bipyridine-5,6-diamine (100 mg, 0.32 mmol) in THF (1.6 mL), Et₃N (88 µL, 0.63 mmol) and carbonyldiimidazole (103 mg, 0.64 mmol) were added sequentially. The reaction mixture was heated at 80°C. After 18 h the solvent was removed under reduced pressure and the crude residue was purified by silica gel flash chromatography (95:5 CH₂Cl₂/MeOH) to give the title compound (83 mg, 77%) as a solid.
δ ¹H-NMR (DMSO): 11.73 (bs, 1H), 11.29 (bs, 1H), 8.50 (s, 2H), 7.50 (s, 1H), 7.42-7.36 (m, 2H), 7.22 (t, 1H), 7.09 (t, 1H).
ESI/MS m/e: 343 ([M+H]⁺, C₁₇H₉F₃N₄O).

### EXAMPLE 4

### 6-(3,5-Difluoropyridin-4-yl)-5-(2-fluorophenyl)-1,3-dihydro-2H-imidazo[4,5-b]pyridin-2-one hydrochloride

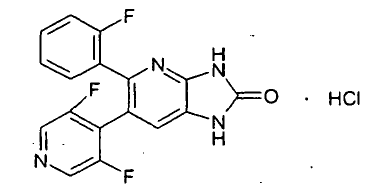

To a solution of 6-(3,5-difluoropyridin-4-yl)-5-(2-fluorophenyl)-1,3-dihydro-2*H*-imidazo[4,5-*b*]pyridin-2-one (Example 3) (75 mg, 0.22 mmol) in dioxane (2 mL) was added 4N HCl (0.15 mL, 0.6 mmol). The mixture was stirred for 2 hours. The mixture was diluted with diethylether (5 mL) and filtered. The solid was dried over solid CaSO₄ under vacuum for 12 h to afford the title salt (72 mg, 87%).
δ ¹H-NMR (DMSO): 11.74 (bs, 1H), 11.27 (s, 1H), 8.46 (bs, 2H), 7.46 (s, 1H), 7.34-7.02 (m, 5H).

### EXAMPLE 5

### 6-(3,5-Difluoropyridin-4-yl)-5-(2-fluorophenyl)-1,3-dihydro-2H-imidazo[4,5-b]pyridin-2-one (tosylate salt)

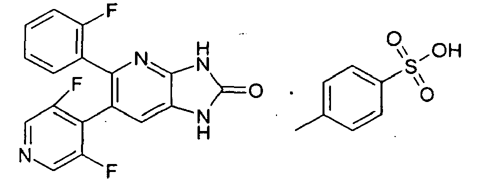

To a solution of 6-(3,5-difluoropyridin-4-yl)-5-(2-fluorophenyl)-1,3-dihydro-2*H*-imidazo[4,5-*b*]pyridin-2-one (Example 3) (80 mg, 0.23 mmol) in dioxane (2 mL) was added *p-*toluenesulfonic acid monohydrate (45 mg, 0.24 mmol). The mixture was stirred for 2 hours. The mixture was diluted with diethylether (5 mL) and filtered. The solid was dried over solid CaSO₄ under vacuum for 12 h to afford the title salt (88 mg, 71%).
δ ¹H-NMR (DMSO): 11.73 (s, 1H), 11.22 (s, 1H), 8.46 (s, 2H), 7.49-7.01 (m, 8H), 6.92 (s, 1H), 2.29 (s, 3H).

### COMPOSITION EXAMPLE 1

50,000 capsules, each containing 100 mg of 6-(3,5-difluoropyrdin-4-yl)-5-(2-fluorophenyl)-1,3-dihydro-2H-imidazo[4,5-b]pyridin-2-one (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of 6-(3,5-difluoropyrdin-4-yl)-5-(2-fluorophenyl)-1,3-dihydro-2H-imidazo[4,5-b]pyridin-2-one (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

## Claims

1. A compound of formula (I) 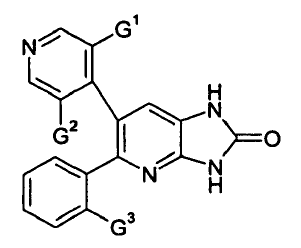 wherein:
G¹ is selected from the groups consisting of fluorine and chlorine atoms;
G² is selected from the groups consisting of hydrogen, fluorine and chlorine atoms; and
G³ is selected from the groups consisting of fluorine and chlorine atoms and pharmaceutically acceptable salts or N-oxides thereof

2. A compound according to claim 1 wherein G³ is a fluorine atom.

3. A compound according to claim 2 wherein G² is a fluorine atom.

4. A compound according to claim 3 wherein G¹ is a fluorine atom.

5. A compound according to claim 1 which is one of:
5-(2-Fluorophenyl)-6-(3-fluoropyrdin-4-yl)-1,3-dihydro-2H-imidazo[4,5-b]pyridin-2-one
6-(3-Chloropyridin-4-yl)-5-(2-fluorophenyl)-1,3-dihydro-2H-imidazo[4,5-b]pyridin-2-one
6-(3,5-Difluoropyridin-4-yl)-5-(2-fluorophenyl)-1,3-dihydro-2H-imidazo[4,5-b]pyridin-2-one
6-(3,5-Difluoropyridin-4-yl)-5-(2-fluorophenyl)-1,3-dihydro-2*H*-imidazo[4,5-*b*]pyridin-2-one hydrochloride
6-(3,5-Difluoropyridin-4-yl)-5-(2-fluorophenyl)-1,3-dihydro-2*H*-imidazo[4,5-*b*]pyridin-2-one (tosylate salt)

6. A pharmaceutical composition comprising a compound as defined in any one of claims 1 to 5 in association with a pharmaceutically acceptable diluent or carrier.

7. A compound according to anyone of claims 1 to 5 for the treatment of a pathological condition or disease susceptible to amelioration by antagonism of the A_{2B} adenosine receptor.

8. A compound according to claim 7, wherein the pathological condition or disease is asthma, bronchoconstriction, allergic diseases, hypertension, atherosclerosis, reperfusion injury, myocardial ischemia, retinopathy, inflammation, gastrointestinal tract disorders, cell proliferation disorders, diabetes mellitus, and/or autoimmune diseases.

9. A compound according to claim 7, wherein the pathological condition or disease is a hepatic disease and wounds.

## Patentansprüche

1. Verbindung von Formel (I) 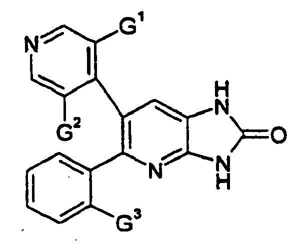 worin:
G¹ ausgewählt ist aus den Gruppen, bestehend aus Fluor- und Chloratomen;
G² ausgewählt ist aus den Gruppen, bestehend aus Wasserstoff-, Fluor- und Chloratomen; und
G³ ausgewählt ist aus den Gruppen, bestehend aus Fluor- und Chloratomen,
und pharmazeutisch verträgliche Salze oder N-Oxide davon.

2. Verbindung nach Anspruch 1, wobei G³ ein Fluoratom ist.

3. Verbindung nach Anspruch 2, wobei G² ein Fluoratom ist.

4. Verbindung nach Anspruch 3, wobei G¹ ein Fluoratom ist.

5. Verbindung nach Anspruch 1, die eine der folgenden ist:
5-(2-Fluorphenyl)-6-(3-fluorpyridin-4-yl)-1,3-dihydro-2H-imidazo[4,5-b]pyridin-2-on
6-(3-Chlorpyridin-4-yl)-5-(2-fluorphenyl)-1,3-dihydro-2H-imidazo[4,5-b]pyridin-2-on
6-(3,5-Difluorpyridin-4-yl)-5-(2-fluorphenyl)-1,3-dihydro-2H-imidazo[4,5-b]pyridin-2-on
6-(3,5-Difluorpyridin-4-yl)-5-(2-fluorphenyl)-1,3-dihydro-2H-imidazo[4,5-b]pyridin-2-on-Hydrochlorid
6-(3,5-Difluorpyridin-4-yl)-5-(2-fluorphenyl)-1,3-dihydro-2H-imidazo[4,5-b]pyridin-2-on (Tosylatsalz)

6. Pharmazeutische Zusammensetzung, umfassend eine Verbindung, wie definiert in einem der Ansprüche 1 bis 5, zusammen mit einem pharmazeutisch verträglichen Verdünnungsmittel oder Trägerstoff.

7. Verbindung nach einem der Ansprüche 1 bis 5 zur Behandlung eines pathologischen Zustandes oder einer Erkrankung, die für Linderung durch Antagonismus des A_{2B}-Adenosinrezeptors empfänglich ist.

8. Verbindung nach Anspruch 7, wobei der pathologische Zustand oder die Erkrankung Asthma, Bronchokonstriktion, allergische Erkrankung, Bluthochdruck, Atherosklerose, Reperfusionsverletzung, myokardiale Ischämie, Retinopathie, Entzündung, Magen-Darm-Trakt-Störungen, Zellproliferationsstörungen, Diabetes mellitus und/oder Autoimmunerkrankungen ist.

9. Verbindung nach Anspruch 7, wobei der pathologische Zustand oder die Erkrankung eine Lebererkrankung und Wunden ist.

## Revendications

1. Composé de formule (I) 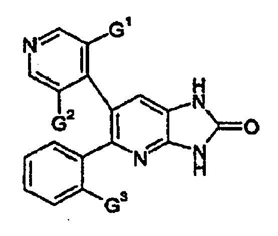 dans laquelle :
G¹ est choisi dans les groupes constitués par des atomes de fluor et des atomes de chlore ;
G² est choisi dans les groupes constitués par des atomes d'hydrogène, des atomes de fluor et des atomes de chlore ; et
G³ est choisi dans les groupes constitués par des atomes de fluor et des atomes de chlore,
et les sels pharmaceutiquement acceptables ou N-oxydes de celui-ci.

2. Composé selon la revendication 1, dans lequel G³ est un atome de fluor.

3. Composé selon la revendication 2, dans lequel G² est un atome de fluor.

4. Composé selon la revendication 3, dans lequel G¹ est un atome de fluor.

5. Composé selon la revendication 1, qui est l'un des composés suivantes :
5-(2-fluorophényl)-6-(3-fluoropyridin-4-yl)-1,3-dihydro-2H-imidazo[4,5-b]pyridin-2-one
6-(3-chloropyridin-4-yl)-5-(2-fluorophényl)-1,3-dihydro-2H-imidazo[4,5-b]pyridin-2-one
6-(3,5-difluoropyridin-4-yl)-5-(2-fluorophényl)-1,3-dihydro-2H-imidazo[4,5-b]pyridin-2-one
chlorhydrate de 6-(3,5-difluoropyridin-4-yl)-5-(2-fluorophényl)-1,3-dihydro-2H-imidazo[4,5-b]pyridin-2-one
6-(3,5-difluoropyridin-4-yl)-5-(2-fluorophényl)-1,3-dihydro-2H-imidazo-[4,5-b]pyridin-2-one (sel de tosylate).

6. Composition pharmaceutique comprenant un composé tel que défini dans l'une quelconque des revendications 1 à 5, en association avec un vecteur ou un diluant pharmaceutiquement acceptables.

7. Composé selon l'une quelconque des revendications 1 à 5, destiné au traitement d'une condition pathologique ou d'une maladie susceptible d'amélioration par antagonisme du récepteur de l'adénosine A_{2B}.

8. Composé selon la revendication 7, dans lequel la condition pathologique ou la maladie est l'asthme, la bronchoconstriction, des maladies allergiques, l'hypertension, l'athérosclérose, une lésion de reperfusion, une ischémie du myocarde, une rétinopathie, une inflammation, des troubles du tractus gastro-intestinal, des troubles de la prolifération cellulaire, le diabète sucré et/ou des maladies auto-immunes.

9. Composé selon la revendication 7, dans lequel la condition pathologique ou la maladie est choisie parmi une maladie hépatique et des blessures.
